# EUROPEAN PATENT SPECIFICATION

(11) **EP 2 252 273 B1**
(45) Date of publication and mention of the grant of the patent: **28.12.2016**
(21) Application number: 09722351.5
(22) Date of filing: 17.03.2009
(51) Int. Cl.: A61K 9/20, A61K 31/4184, A61K 31/549, A61K 45/06, A61P 9/12

(54) **SOLID PHARMACEUTICAL COMPOSITION COMPRISING A NON-PEPTIDE ANGIOTENSIN II RECEPTOR ANTAGONIST AND A DIURETIC**
PHARMAZEUTISCHE FESTSTOFFZUSAMMENSETZUNG MIT EINEM NICHT-PEPTIDISCHEN ANGIOTENSIN-II-REZEPTORANTAGONISTEN UND EINEM DIURETIKUM
COMPOSITION PHARMACEUTIQUE SOLIDE INCLUANT UN ANTAGONISTE NON PEPTIDIQUE DU RÉCEPTEUR DE L'ANGIOTENSINE II ET UN DIURÉTIQUE

(30) Priority: 19.03.2008 IN DE07002008
(43) Date of publication of application: 24.11.2010
(73) Proprietor: ratiopharm GmbH, 89079 Ulm (DE)
(72) Inventor: MHASE, Sunil, R., Ahmednagar 413 711, Maharashtra (IN); GAT, Ganesh, V., Pune 411 052, Maharashtra (IN); HUSSAIN, Jawed, Aurangabad 431 003, Maharashtra (IN)
(74) Representative: Teipel, Stephan
(86) International application number: PCT/EP2009/001966
(87) International publication number: WO 2009/115301

(56) References cited:
- WO-A-03/059327
- WO-A-2005/082329
- WO-A-2007/144175
- WO-A-2009/058950
- WO-A1-2006/063737
- WO-A2-2007/060170
- US-A1- 2005 186 274
- ROWE R C; SHESKEY P J; OWEN S C (ED): "Handbook of Pharmaceutical Excipients" 2006, PHARMACEUTICAL PRESS AND AMERICAN PHARMACISTS ASSOCIATION , WASHINGTON, DC , XP002528077 page 346 - page 349 page 718 - page 721 page 348, left-hand column, 11. Stability and Storage Conditions page 719, right-hand column, 11. Stability and Storage Conditions
- ROWE R C ET AL: "POVIDONE", 1 January 2006 (2006-01-01), HANDBOOK OF PHARMACEUTICAL EXCIPIENTS, PHARMACEUTICAL PRESS [U.A.], GB, PAGE(S) I-II, XP003024361, ISBN: 978-1-58212-058-4

## Description

### Field of the invention

The present invention relates to a solid pharmaceutical composition comprising at least two layers, wherein the first layer contains a non-peptide angiotensin II receptor antagonist or a pharmaceutically acceptable salt thereof in a dissolving matrix and the second layer contains a diuretic or a pharmaceutically acceptable salt thereof.

### Background of the invention

Non-peptide angiotensin II receptor antagonists are known for the treatment of hypertension. Angiotensin II is a principal pressor agent of the renin-angiotensin-system. Angiotensin II is formed from Angiotensin I, in a reaction catalyzed by the Angiotensin converting enzyme (ACE, kinenase II). Effects mediated by the AT₁ receptor include vasoconstriction, stimulation of synthesis and release of aldosterone, cardiac stimulation and renal re-absorption of sodium. Angiotensin II receptor antagonists block the vasoconstrictor and aldosterone secreting effects of Angiotensin II by selectively blocking the binding of Angiotensin II to the AT₁ receptor in many tissues, such as vascular smooth muscles and the adrenal gland. Their action is therefore independent of the pathways of Angiotensin II synthesis. Among the known angiotensin II receptor antagonists Telmisartan is indicated for the treatment of hypertension. Telmisartan is sold under the trade name *Micardis^{®}* by Boehringer Ingelheim.

Telmisartan, also known as 4'-[(1,4'-dimethyl-2'-n-propyl-[2,6'-bi-1H-benzimidazol]-1'-yl)methyl]-[1,1'-biphenyl]-2-carboxylic acid, has the following structural formula I:

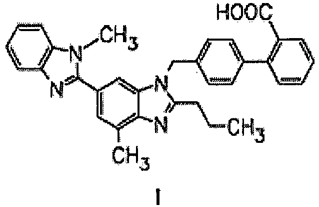

Telmisartan is generally manufactured and supplied in the free acid form. It is characterized by its very poor solubility in aqueous systems at the physiological pH range of the gastrointestinal tract of between pH 1 - 7.

WO 00/43370 discloses that crystalline Telmisartan exists in two polymorphic forms having different melting points. Under the influence of heat and humidity, the lower melting polymorph B transforms irreversibly into the higher melting polymorph A. Depending on temperature, pH, humidity and residence time mixtures of polymorph A and B can be obtained.

Diuretics are orally administered in the treatment of edema and hypertension. A well known diuretic is Hydrochlorothiazide (HCTZ), a thiazide diuretic. The chemical name of HCTZ is 6-chloro-3,4-dihydro-2H-1,2,4-benzothiadiazine-7-sulfonamide-1,1-dioxide having the following structure (II).

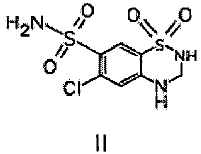

The combination therapy of a non-peptide angiotensin II receptor antagonist such as Telmisartan or a pharmaceutically acceptable salt thereof with a diuretic such as HCTZ is expected to show synergistic therapeutic efficacy in the treatment of hypertension.

Investigations therefore were made to provide a fixed dose drug combination comprising a non-peptide angiotensin II receptor antagonist or a pharmaceutically acceptable salt thereof Such combination drug should display an immediate drug release profile combined with adequate stability.

A fixed dose combination of drugs intended for immediate release can be prepared by either making a powder mixture or a co-granulate of the two active ingredients with the necessary excipients, normally keeping the basic formulation of the corresponding mono-drug preparation and simply adding the second drug component.

However, particularly with a combination of Telmisartan or a pharmaceutically acceptable salt thereof and HCTZ, this approach was not feasible due to the incompatibility of HCTZ with basic compounds such as, e.g., meglumine (N-methyl-D-glucamine) which is a component of conventional Telmisartan formulations.

Several galenical approaches to overcome the incompatibility problem have been described. An approach is to coat the HCTZ particle in a fluidized-bed granulator with a polymer solution containing water soluble polymers like hydroxypropylcellulose, hydroxypropylmethylcellulose or polyvinylpyrrolidone, thereby reducing the contact surface area of the HCTZ particles with the Telmisartan formulation during mixing and compressing. However, by these means it was not possible to reduce the surface contact area of HCTZ with the Telmisartan formulation in a compressed tablet to a degree sufficient to achieve the desired prolonged shelf life.

Another approach is to produce separate film-coated tablets of Telmisartan or a pharmaceutically acceptable salt thereof and of HCTZ in such a size and shape that these could be filled into a capsule. By dividing the doses into two or more single small tablets for Telmisartan or a pharmaceutically acceptable salt thereof and into one or two small tablets of HCTZ a capsule size of 1 or 0 long could be filled. With this approach the drug dissolution rate of Telmisartan or a pharmaceutically acceptable salt thereof is expected to be reduced compared to the single entities due to a lag-time effect of the large capsule shells. Furthermore, with regard to patient's compliance a size 0 long capsule is not deemed convenient.

A third approach is the preparation of a fixed dose combination drug comprising Telmisartan or a pharmaceutically acceptable salt thereof and a diuretic by means of pharmaceutical tablet comprising a first layer containing Telmisartan or a pharmaceutically acceptable salt thereof and a second layer containing a diuretic and/or optionally a dummy layer (without the active ingredients Telmisartan and HCTZ) in between first and second layer.

WO 2003/059327 discloses a bilayer pharmaceutical tablet comprising a first layer containing Telmisartan in substantially amorphous form in a dissolving tablet matrix and a second layer containing a diuretic in a disintegrating tablet matrix. This bilayer tablet structure overcomes the stability problem caused by the incompatibility of a diuretics like HCTZ with basic constituents of the Telmisartan formulation. At the same time such a formulation provides for immediate release of the diuretic from the fast disintegrating matrix.

However, it has now been found by the present inventors that the use of disintegrants in the fast disintegrating tablet matrix known from WO 2003/059327 exhibits several drawbacks. Tablets with the fast disintegrating matrix tend to be hygroscopic and, therefore, need to be packaged using a moisture-proof packaging material such as aluminium foil blister packs, or polypropylene tubes and HDPE bottles which preferably contain a desiccant.

Moreover, the disintegrant results in tablets which have a tendency to stick to the patients tongue due to rapid absorption, dehydrating the moist surface thereby causing adhesion. This problem could be overcome by an outer layer coating which, however, in turn means another process step and increased production cost, as well as causing slower and/or lagged dissolution of the actives.

Furthermore, coating is inevitable in cases in which an unpleasant-tasting active ingredient is used in order to guarantee a flavour-concealing or taste-masking. According to US 6,136,347, a disadvantage of disintegrant containing recipes, however, is that considerable amounts of coating have to be applied in order to guarantee a flavour-concealing. This leads to a prolongation of the encapsulation process period and an increase in costs.

WO 2005/082329 discloses a solid dosage form comprising a core and a coating layer, wherein the core contains valsartan and the coating layer contains HCTZ. The core additionally contains crosspovidone, carboxymethyl cellulose calcium and carboxymethyl cellulose sodium.

US 2005/0186274 discloses a pharmaceutical tablet comprising a first layer of telmisartan in a dissolving tablet matrix and its second layer of ramipril in a disintegrating tablet matrix.

WO 2006/063737 discloses a bilayer tablet consisting of a first tablet layer comprising telmisartan in a dissolving tablet matrix and a separate second tablet layer comprising the active ingredient HCTZ in a disintegrating tablet matrix.

WO 2007/060170 discloses a bilayer pharmaceutical tablet comprising a first layer containing telmisartan dispersed in a dissolving matrix and a second layer containing a diuretic in a disintegrating tablet matrix.

### Object of the invention

It was, therefore, an object of the present invention to provide a fixed dose drug combination comprising a non-peptide angiotensin II receptor antagonist and a diuretic which does not exhibit the above described drawbacks. In particular, the combination drug should display an immediate drug release profile combined with adequate stability.

### Summary of the invention

It has now surprisingly been found that the above described problems associated with conventional approaches in the preparation of fixed dose combination drugs comprising a non-peptide angiotensin II receptor antagonist and a diuretic can be overcome by incorporating the diuretic in a dissolving matrix without the use of any disintegrants. This finding is particularly unexpected since it was known from the above described prior art documents that the intended immediate release of the diuretic requires the incorporation of this active ingredient in a disintegrating tablet matrix.

Thus, the present invention relates to a solid pharmaceutical composition comprising at least two layers, wherein the first layer contains a non-peptide angiotensin II receptor antagonist or a pharmaceutically acceptable salt thereof in a dissolving matrix and the second layer contains a diuretic or a pharmaceutically acceptable salt thereof in a dissolving matrix, the pharmaceutical composition does not contain any disintegrant, and the non-peptide angiotensin II receptor antagonist is selected from the group consisting of Telmisartan, Losartan, Irbesartan, Valsartan, Eprosartan, Candesartan, Candesartan Cilexetil, Olmesartan and Olmesartan Medoxomil and the Telmisartan or a pharmaceutically acceptable salt thereof is in at least 90 % amorphous form, based on the total amount of Telmisartan or the pharmaceutically acceptable salts thereof.

The solid pharmaceutical composition of the present invention, preferably in the form of a tablet, provides a pH independent dissolution of the non-peptide angiotensin II receptor antagonist, in particular of the poorly water soluble Telmisartan or pharmaceutically acceptable salts thereof, thereby facilitating the dissolution of the drug at a physiological pH level and also provides an immediate release of a diuretic from the dissolving tablet matrix. At the same time the tablet structure overcomes the stability problem caused by the incompatibility of diuretics like HCTZ with basic excipients of the Telmisartan formulation, e.g. meglumine or alkali metal hydroxides like NaOH or KOH. Omitting disintegrants in any of the layers of the composition avoids the necessity to coat the tablet in order to prevent tongue sticking and facilitates taste masking.

### Detailed description of the invention

The present invention provides a solid pharmaceutical composition comprising at least two layers, wherein the first layer contains a non-peptide angiotensin II receptor antagonist or a pharmaceutically acceptable salt thereof in a dissolving matrix and the second layer contains a diuretic or a pharmaceutically acceptable salt thereof in a dissolving matrix.

The non-peptide angiotensin II receptor antagonists is selected from the group consisting of Telmisartan, Losartan, Irbesartan, Valsartan, Eprosartan, Candesartan, Candesartan Cilexetil, Olmesartan and Olmesartan Medoxomil. Preferably, the non-peptide angiotensin II receptor antagonist is Telmisartan.

The diuretic in the solid pharmaceutical composition of the present invention can be selected from the group consisting of Hydrochlorothiazide, Xipamide, Acetazolamid, Chlortalidon, Furosemid, Piretanid and Torasemid. Hydrochlorothiazide is preferred.

Any pharmaceutically acceptable salt of the active ingredients can be employed. Corresponding salts are known to the person skilled in the art. Examples of pharmaceutically acceptable salts of Telmisartan are salts which are formed in the reaction of Telmisartan with a base, such as Telmisartan Sodium, Telmisartan Potassium and Telmisartan Meglumiate.

The term "dissolving matrix" refers to a pharmaceutically base formulation having immediate release (fast dissolution) characteristics that readily dissolves in a physiological aqueous medium. A dissolving matrix is distinguishable from a disintegrating matrix in that the dissolving matrix readily dissolves but does not readily swell and disintegrate in a physiological aqueous medium. In particular, a dissolving matrix can be distinguished from a disintegrating matrix in that it does not contain any disintegrant.

The solid pharmaceutical composition of the present composition does not contain any disintegrant.

Compounds which are considered as disintegrants in the field of solid pharmaceutical preparations are known to the person skilled in the art. In particular, alginic acid, bentonite, calcium carboxymethylcellulose, sodium carboxymethylcellulose, Crospovidon, sodium carboxymethyl starch, sodium starch glycolate and starch, in particular dried corn starch, are considered as disintegrants. Soy polysaccharides can also be considered as disintegrants. Most preferably, the solid pharmaceutical composition of the present invention does not contain any sodium starch glycolate.

The present solid pharmaceutical composition preferably is a tablet. In one embodiment the tablet is a bilayer tablet. In a further embodiment of the present invention the tablet comprises more than two layers for example by incorporating a "dummy layer" between the first and the second layer. The "dummy layer" in this embodiment is a layer without active pharmaceutical ingredients. If desired or necessary the tablet may also comprise four or more layers.

The two or more layers of the solid pharmaceutical composition of the present invention can be in the form of adjacent parallel layers or any other configuration, for example one layer enclosing the other layer. The at least two layers and optional further layers can also be repeated for two or several times so as to form a sandwich-like structure.

If the composition is in the form of a tablet, this tablet preferably does not comprise any coating. It is, however, nevertheless possible that the tablet has a coating. In that case any usual tablet coating can be applied.

The preferred non-peptide angiotensin II receptor antagonist in the first layer of the present solid pharmaceutical composition is Telmisartan or a pharmaceutically acceptable salt thereof. This active ingredient is present in substantially amorphous form. In this regard the term "in substantially amorphous form" relates to Telmisartan or a pharmaceutically acceptable salt thereof comprising amorphous Telmisartan or a pharmaceutically acceptable salt thereof in a proportion of at least 90%, most preferably at least 95% of the total amount of Telmisartan or the pharmaceutically acceptable salt thereof, as determined by X-ray (powder) diffraction measurement. Telmisartan in substantially amorphous form can be obtained as described in WO 2003/059327.

In a particularly preferred embodiment the solid pharmaceutical composition of the present invention comprises Telmisartan or a pharmaceutically acceptable salt thereof in the first layer and Hydrochlorothiazide in the second layer.

In the first layer the solid pharmaceutical composition of the present invention can additionally comprise a basic compound. Examples of suitable basic compounds are Bronsted bases, such as alkali metal carbonates, in particular sodium carbonate or potassium carbonate, alkali metal bicarbonates, in particular sodium bicarbonate, or alkali metal hydroxides, in particular sodium hydroxide or potassium hydroxide, basic amino acids, in particular arginine and lysine, and meglumine (N-methyl-D-glucamine). Sodium hydroxide and meglumine are preferred basic agents.

The dissolving matrix of the first layer may comprise in addition to the basic compound a water soluble diluent and, optionally, other excipients and adjuvants as known to the person skilled in the art.

Examples of suitable water-soluble diluents are carbohydrates such as monosaccharides like glucose; oligosaccharides like sucrose, anhydrous lactose, and lactose monohydrate; and sugar alcohols like sorbitol, mannitol, erythrol, and xylitol. Sorbitol and mannitol are preferred diluents.

The other excipients and adjuvants, if used, are preferably selected from binders, carriers, fillers, lubricants, flow control agents, crystallization retarders, solubilizers, coloring agents, pH control agents, specific examples of which are given below. The excipients and/or adjuvants for the first layer are preferably chosen such that a non-acidic, fast dissolving tablet matrix is obtained.

The first layer comprising Telmisartan as an example may be produced by performing the steps of:
1) Preparing spray solution by dissolving at least one basic compound, binder and Telmisartan in a suitable solvent like purified water.
2) Granulating sifted sorbitol, mannitol, and meglumine in a fluid bed granulator with the spray solution followed by drying and screening step(s) to obtain Telmisartan granules.
3) Blending of the Telmisartan granules with sifted talc and magnesium stearate in a blender to obtain the final blend and compressing the final blend to form the first tablet layer.

The second dissolving matrix of the diuretic layer preferably comprises one or more fillers, a binder or polymer, a lubricant and, optionally, other excipients and adjuvants as known to the person skilled in the art.

Preferred fillers are for example selected from the group consisting of mannitol, erythritol, lactose, sucrose, calcium hydrogen phosphate, sorbitol, and xylitol. Particularly preferred are mannitol and/or lactose monohydrate.

Preferred binders are for example selected from the group consisting of copolymers of vinylpyrrolidone with other vinyl derivatives (Copovidone), hydroxypropyl methylcellulose, methylcellulose, hydroxypropyl cellulose (Klucel LF, Klucel LXF, Klucel EF, Klucel EXF, Klucel JF, Klucel JXF, Klucel GF, Klucel GXF, Klucel MF, Klucel HF). Particularly preferred are hydroxypropyl cellulose (Klucel LF) and/or hydroxypropyl methylcellulose and/or mannitol.

Preferred lubricants are for example sodium stearyl fumarate and/or magnesium stearate. The other excipients and adjuvants, if used, are for example selected from:
- diluents and carriers such as cellulose derivatives like hydroxymethylcellulose, hydroxyethylcellulose, hydroxypropylcellulose and hydroxypropyl methylcellulose, dibasic calcium phosphate, etc.;
- lubricants such as stearic acid, magnesium stearate, sodium stearyl fumarate, glycerol tribehenate, etc.;
- flow control agents such as colloidal silica, talc, etc.;
- crystallization retarders;
- solubilizers such as Pluronic, etc.;
- coloring agents, including dyes and pigments such as Iron Oxide Red or Yellow, titanium dioxide, talc, etc.;
- pH control agents such as citric acid, tartaric acid, fumaric acid, sodium citrate, dibasic calcium phosphate, dibasic sodium phosphate, etc.;
- surfactants and emulsifiers such as Pluronic, polyethylene glycols, polyethoxylated, and hydrogenated castor oil, etc.;
- antioxidants,
   and mixtures of two or more of these excipients and/or adjuvants.

The layers can be differentiated by using different colors.

The second layer comprising hydrochlorothiazide as an example may be produced by wet granulation and/or direct compression comprising the steps of:

### A) Wet granulation method:

1) Preparing a binder solution by dissolving a binding agent in water.
2) Mixing and wet granulation of sifted Hydrochlorothiazide, fillers, and optionally coloring agent(s) with the aqueous binder solution, followed by drying and screening/sifting to obtain Hydrochlorothiazide granules.
3) Blending of the Hydrochlorothiazide granules with sifted talc and magnesium stearate in a blender to obtain the final blend and compressing the final blend to form the second tablet layer.

### B) Direct compression method:

1) Mixing of sifted Hydrochlorothiazide, lactose monohydrate, and coloring agent(s) like Iron oxide red in a bin blender.
2) Blending of the above described mixture with sifted talc and Sodium stearyl fumarate to obtain the final blend and compressing the final blend to form the second tablet layer.

The optional dummy layer preferably comprises one or more fillers, a binder or polymer, a lubricant and, optionally, other excipients and adjuvants as described above. The said dummy layer blend may be prepared by wet granulation method and/or direct compression method.

The above prepared separate tablet layers are compressed into tablets comprising more than one layer.
Figure 1 illustrates the dissolution profile of Telmisartan from the tablets manufactured according to examples 1 and 4.
Figure 2 illustrates the dissolution profile of Hydrochlorothiazide from the tablets manufactured according to examples 1 and 4.

In order to further illustrate the present invention, the following non-limiting examples are given.

### Examples

### Examples 1, 2 and 3:

Examples 1, 2 and 3 describe excipients and wet granulation procedures for the preparation of a Telmisartan layer and a Hydrochlorothiazide layer of tablets according to the present invention. The compounds of the tablets are given in Table 1.

**Table 1**

| **Telmisartan and Hydrochlorothiazide Tablet 80/12.5 mg** | | | | |
|---|---|---|---|---|
| **Sr. No.** | **Ingredients** | **Example 1** | **Example 2** | **Example 3** |
| | | **mg/tablet** | **mg/tablet** | **mg/tablet** |
| **Telmisartan Phase** | | | | |
| 1 | Telmisartan | 80 | | |
| 2 | Sodium Hydroxide | 6.72 | | |
| 3 | Hydroxy propyl methyl cellulose | 14.4 | | |
| 4 | Sorbitol | 56.74 | | |
| 5 | Mannitol | 274.14 | | |
| 6 | Meglumine | 21.12 | | |
| 7 | Talc | 12 | | |
| 8 | Magnesium stearate | 4.88 | | |
| 9 | Purified water* | q.s. | | |
| **Telmisartan Layer weight** | | 470 mg | | |

| **Hydrochlorothiazide Phase** | | | | |
|---|---|---|---|---|
| 10 | Hydrochlorothiazide | 12.5 | 12.5 | 12.5 |
| 11 | Lactose monohydrate | 175.1 | 170 | 88 |
| 12 | Hydroxy propyl cellulose (Klucel LF) | 3 | - | - |
| 13 | Hydroxy propyl methyl cellulose | - | 8 | - |
| 14 | Mannitol | - | - | 90 |
| 15 | Red iron oxide | 0.4 | 0.5 | 0.5 |
| 16 | Talc | 6 | 6 | 6 |
| 17 | Magnesium stearate | 3 | 3 | 3 |
| 18 | Purified water* | q.s | q.s | q.s |
| **HCTZ Layer weight** | | 200 mg | 200 mg | 200 mg |
| **Total tablet weight** | | 670 mg | 670 mg | 670 mg |

| | | | | |
|---|---|---|---|---|
| *- Volatile component q.s - Quantity sufficient. | | | | |

### Manufacturing Process :

### A) Telmisartan phase blend preparation (Examples 1, 2, and 3):

The spray solution was prepared by dissolving sodium hydroxide, telmisartan, and hydroxyl propyl methyl cellulose in purified water. Sifted sorbitol, mannitol, and meglumine were briefly mixed and granulated with the spray solution in a fluid bed granulator followed by screening and/or sifting.

The Screened and/or sifted granules were mixed with sifted talc and magnesium stearate in a blender to obtain the final blend.

### B) Hydrochlorothiazide phase blend preparation (Examples 1 and 2):

The binder solution was prepared by dissolving hydroxyl propyl cellulose (Klucel LF) or Hydroxy propyl methyl cellulose in purified water. A co-sifted mixture of Hydrochlorothiazide, lactose monohydrate and iron oxide red was placed, homogeneously mixed and then granulated with binder solution in a high shear mixer. The resulting wet granules were dried, screened and/or sifted, mixed with sifted talc and magnesium stearate in a blender to obtain the final blend.

### C) Hydrochlorothiazide phase blend preparation (Example 3):

Co-sifted Hydrochlorothiazide, lactose monohydrate, mannitol and iron oxide red were homogeneously mixed in a high shear mixer. The resulting mixture was granuled by adding purified water. The resulting wet granules were dried, screened and/or sifted, mixed with sifted talc and magnesium stearate in a blender to obtain the final blend.

### D) Compression:

The said tablets comprising of more than one layer were manufactured by compressing the above prepared blend of Telmisartan and Hydrochlorothiazide using suitable compression machine.

### Example 4:

Examples 4 describes excipients and a wet granulation procedure for the preparation of the Telmisartan layer and a direct compression procedure for the Hydrochlorothiazide layer of a tablet according to the present invention. The composition of the tablets is shown in Table 2

**Table 2**

| **Telmisartan and Hydrochlorothiazide Tablet 80/12.5 mg** | | |
|---|---|---|
| **Sr. No.** | **Ingredients** | **Example 4** |
| | | **mg/tablet** |
| **Telmisartan Phase** | | |
| 1 | Telmisartan | 80 |
| 2 | Sodium Hydroxide | 6.72 |
| 3 | Hydroxy propyl methyl cellulose | 14.4 |
| 4 | Sorbitol | 56.74 |
| 5 | Mannitol | 274.14 |
| 6 | Meglumine | 21.12 |
| 7 | Talc | 12 |
| 8 | Magnesium stearate | 4.88 |
| 9 | Purified water* | q.s. |
| **Telmisartan Layer weight** | | 470 mg |

| **Hydrochlorothiazide Phase** | | |
|---|---|---|
| 10 | Hydrochlorothiazide | 12.5 |
| 11 | Lactose monohydrate | 185.5 |
| 14 | Red iron oxide | 0.5 |
| 15 | Sodium stearyl fumarate | 1.5 |
| **HCTZ Layer weight** | | 200 mg |
| **Total tablet weight** | | 670 mg |

| | | |
|---|---|---|
| *- Volatile component q.s - Quantity sufficient. | | |

### Manufacturing Process:

### A) Telmisartan phase blend preparation (Example 4):

A similar manufacturing process as described in example 1, 2 and 3 was used for manufacturing of the telmisartan layer.

### B) Hydrochlorothiazide phase Blend Preparation (Example 4):

Co-sifted Hydrochlorothiazide, lactose monohydrate and iron oxide red were homogeneously mixed in a high shear mixer. The resulting mixture was then lubricated by adding and mixing of sifted talc and magnesium stearate to obtain the final blend.

### C) Compression:

The said tablets comprising of more than one layer were manufactured by compressing the above prepared blend of Telmisartan and Hydrochlorothiazide using a suitable compression machine.

Telmisartan and Hydrochlorothiazide tablets comprising 40 mg Telmisartan and 12.5 mg Hydrochlorothiazide may be manufactured by using the above prepared blend of Telmisartan layer dose proportionally and the Hydrochlorothiazide layer blend as such. Other strengths and/or strength combinations such as for example 80 / 12.5 mg, 160 / 25 mg or 160 / 12.5 mg can be prepared in a analogous manner.

### Example 5

In vitro dissolution testing of the Telmisartan and Hydrochlorothiazide combination tablets of examples 1 to 4 were performed. For the Telmisartan component, the dissolution test were conducted using Apparatus II (Paddle method) as described in the United State Pharmacopoeia XXI/National Formulary XVI. The dissolution test was conducted in a USP type II apparatus at a paddle speed of 75 rpm, at a temperature of 37 °C, in 900 ml of a buffer at pH 7.5.

For the Hydrochlorothiazide component, the dissolution procedure described in the USP was used. The dissolution test is performed in 900 ml of 0.1 N HCl acid using USP type I Apparatus (Basket) at 100 rpm. The proposed specification for the testing of HCTZ in the tablet is Q=60% after 60 minutes.

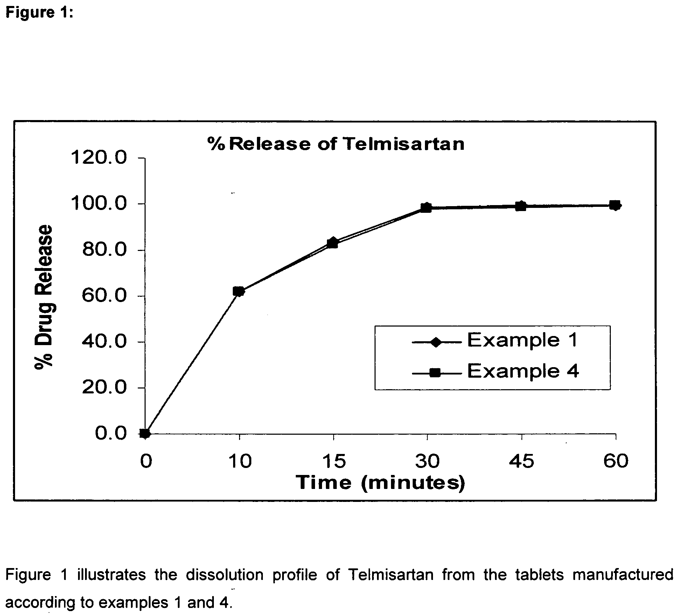

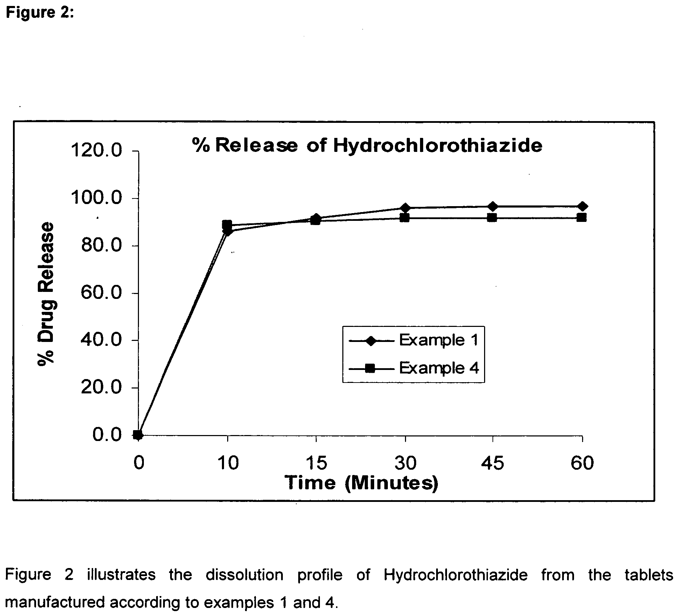

The results are shown in Figures 1 and 2.

## Claims

1. A solid pharmaceutical composition comprising at least two layers, wherein the first layer contains a non-peptide angiotensin II receptor antagonist or a pharmaceutically acceptable salt thereof in a dissolving matrix and the second layer contains a diuretic or a pharmaceutically acceptable salt thereof in a dissolving matrix, the pharmaceutical composition does not contain any disintegrant, and the non-peptide angiotensin II receptor antagonist is selected from the group consisting of Telmisartan, Losartan, Irbesartan, Valsartan, Eprosartan, Candesartan, Candesartan Cilexetil, Olmesartan and Olmesartan Medoxomil and the Telmisartan or a pharmaceutically acceptable salt thereof is in at least 90 % amorphous form, based on the total amount of Telmisartan or the pharmaceutically acceptable salts thereof.

2. The solid pharmaceutical composition of claim 1, wherein the diuretic is selected from the group consisting of Hydrochlorothiazide, Xipamide, Acetazolamid, Chlortalidon, Furosemid, Piretanid and Torasemid.

3. The solid pharmaceutical preparation of any of the preceding claims which is a tablet, preferably a bilayer tablet.

4. The solid pharmaceutical preparation of claim 1, wherein the diuretic is Hydrochlorothiazide.

5. The solid pharmaceutical composition of any of claims 1 or 4, wherein the first layer additionally comprises a basic compound, preferably meglumine.

6. The solid pharmaceutical composition of any of claims 1 or 3 to 5 which does not comprise any coating.

## Patentansprüche

1. Pharmazeutische Feststoffzusammensetzung, umfassend zumindest zwei Schichten, wobei die erste Schicht einen Nicht-Peptid-Angiotensin II-Rezeptorantagonisten oder ein pharmazeutisch akzeptables Salz davon in einer sich auflösenden Matrix enthält und die zweite Schicht ein Diuretikum oder ein pharmazeutisch akzeptables Salz davon in einer sich auflösenden Matrix enthält, die pharmazeutische Zusammensetzung keinen Lösungsvermittler enthält und der Nicht-Peptid-Angiotensin II-Rezeptorantagonist ausgewählt ist aus der Gruppe, bestehend aus Telmisartan, Losartan, Irbesartan, Valsartan, Eprosartan, Candesartan, Candesartancilexetil, Olmesartan und Olmesartanmedoxomil, und das Telmisartan oder ein pharmazeutisch akzeptables Salz davon zumindest zu 90 % in amorpher Form vorliegt, basierend auf der Gesamtmenge an Telmisartan oder den pharmazeutisch akzeptablen Salzen davon.

2. Pharmazeutische Feststoffzusammensetzung nach Anspruch 1, wobei das Diuretikum ausgewählt ist aus der Gruppe, bestehend aus Hydrochlorthiazid, Xipamid, Acetazolamid, Chlortalidon, Furosemid, Piretanid und Torasemid.

3. Pharmazeutisches Feststoffpräparat nach einem der vorhergehenden Ansprüche, das eine Tablette, bevorzugt eine zweischichtige Tablette ist.

4. Pharmazeutisches Feststoffpräparat nach Anspruch 1, wobei das Diuretikum Hydrochlorthiazid ist.

5. Pharmazeutische Feststoffzusammensetzung nach einem der Ansprüche 1 oder 4, wobei die erste Schicht zusätzlich eine basische Verbindung, bevorzugt Meglumin, umfasst.

6. Pharmazeutische Feststoffzusammensetzung nach einem der Ansprüche 1 oder 3 bis 5, die keine Beschichtung umfasst.

## Revendications

1. Composition pharmaceutique solide comprenant au moins deux couches, dans laquelle la première couche contient un antagoniste non peptidique des récepteurs de l'angiotensine II ou un sel pharmaceutiquement acceptable de celui-ci dans une matrice de dissolution et la deuxième couche contient un diurétique ou un sel pharmaceutiquement acceptable de celui-ci dans une matrice de dissolution, la composition pharmaceutique ne contient pas de délitant, et l'antagoniste non peptidique des récepteurs de l'angiotensine II est choisi dans le groupe constitué du Telmisartan, du Losartan, de l'Irbesartan, du Valsartan, de l'Eprosartan, du Candesartan, du Candesartan Cilexetil, de l'Olmesartan et de l'Olmesartan Medoxomil et le Telmisartan ou un sel pharmaceutiquement acceptable de celui-ci se présente sous une forme au moins 90 % amorphe, sur la base de la quantité totale du Telmisartan ou des sels pharmaceutiquement acceptables de ceux-ci.

2. Composition pharmaceutique solide selon la revendication 1, dans laquelle le diurétique est choisi dans le groupe constitué de l'Hydrochlorothiazide, du Xipamide, de l'Acetazolamid, du Chlortalidon, du Furosemid, du Piretanid et du Torasemid.

3. Préparation pharmaceutique solide selon l'une quelconque des revendications précédentes qui est un comprimé, de préférence un comprimé bicouche.

4. Préparation pharmaceutique solide selon la revendication 1, dans laquelle le diurétique est l'Hydrochlorothiazide.

5. Composition pharmaceutique solide selon l'une quelconque des revendications 1 ou 4, dans laquelle la première couche comprend en outre un composé basique, de préférence de la méglumine.

6. Composition pharmaceutique solide selon l'une quelconque des revendications 1 ou 3 à 5 qui ne comprend pas d'enrobage.
